# DEMANDE DE BREVET EUROPEEN

(11) **EP 1 249 606 A1**
(43) Date de publication de la demande: **16.10.2002**
(21) Numéro de dépôt: 01810354.9
(22) Date de dépôt: 10.04.2001
(51) Int. Cl.: F04B 43/12, A61M 5/142

(54) **Pompe à cassette programmable pour l'injection de médicaments**

(71) Demandeur: Precimedix S.A., 2300 La Chaux-de-Fonds (CH)
(72) Inventeur: Ray, Claude, 2205 Montezillon (CH)
(74) Mandataire: Gresset, Jean

(57) **Abrégé**

L'invention concerne une pompe programmable pour l'injection de médicaments, comportant deux parties accouplables de manière amovible, soit:
- une cassette (12) contenant un liquide à injecter, et
- un module pompe doté de moyens d'actionnement pour faire passer le liquide de la cassette vers l'extérieur.

La cassette (12) comporte une mémoire électronique (66) dans laquelle ont été préalablement enregistrées des données relatives au programme d'injection prescrit et en ce que, lorsque les deux parties sont accouplées, lesdits moyens d'actionnement se trouvent électriquement connectés à la mémoire et répondent aux données qu'elle contient pour injecter le liquide selon le programme désiré.

## Description

La présente invention se rapporte aux pompes miniaturisées pour l'injection de médicaments selon un programme prescrit. Elle concerne, plus particulièrement, une pompe médicale programmable formée d'un module pompe et d'une cassette contenant le médicament, qui s'accouplent de manière amovible.

L'invention concerne également un équipement destiné à la programmation d'une telle pompe.

Les pompes miniaturisées à usage médical sont connues depuis plusieurs années. Légères et de faibles dimensions, elles sont portées par le patient de façon discrète et sans inconfort et permettent de lui administrer, par voie sous-cutanée ou intraveineuse, en continu ou selon un programme déterminé, des quantités contrôlées de solutions médicamenteuses, sans qu'il soit, pour autant, alité et relié à un appareil encombrant, coûteux et bruyant.

De telles pompes sont, souvent, de type péristaltique rotatif. Leur principe consiste à disposer d'un tuyau en matière plastique déformable relié à un réservoir contenant la solution de médicament et à l'écraser localement contre une pièce d'appui de forme arrondie au moyen de galets presseurs montés sur un rotor entraîné par un moteur agissant par l'intermédiaire d'un train d'engrenages. Le liquide est ainsi aspiré du réservoir et poussé vers le sortie pour être injecté dans le corps du patient.

Le brevet FR 2 753 235 et le document EP 01810096.6, par exemple, décrivent des pompes à cassette de ce type.

Lors de la conception de ces pompes, il est particulièrement important de se préoccuper de leur facilité de programmation. Destinées à être utilisées en milieu non hospitalier, elles doivent, en effet, pouvoir être mises en service, de manière sûre, par tout personnel soignant, voire par le patient lui-même, sans qu'il ait reçu une formation spéciale ou doive lire un long et compliqué mode d'emploi. Or, ces exigences de convivialité et de sécurité sont rarement satisfaites par les pompes actuellement sur le marché. Le risque est alors qu'elles restent un équipement d'hôpital réservé à quelques infirmières, ce qui leur fait perdre une grande partie de leur intérêt.

Le but de la présente invention est donc de fournir une pompe à cassette immédiatement utilisable par toute personne, sans nécessiter aucune formation ou connaissance particulière.

De façon plus précise, l'invention concerne une pompe programmable pour l'injection de médicaments, comportant deux parties accouplables de manière amovible, soit:
- une cassette contenant un liquide à injecter, et
- un module pompe doté de moyens d'actionnement pour faire passer le liquide de la cassette vers l'extérieur,
caractérisée en ce que la cassette comporte une mémoire électronique dans laquelle ont été préalablement enregistrées des données relatives au programme d'injection prescrit et en ce que, au moment de l'accouplement des deux parties, les moyens d'actionnement se trouvent électriquement connectés à la mémoire et répondent aux données qu'elle contient pour injecter le liquide selon le programme désiré.

De préférence, le module pompe est de type péristaltique. Il comporte un boîtier et, à l'intérieur de celui-ci, un rotor muni de galets presseurs, un moteur pas à pas pour l'entraînement du rotor et un microprocesseur pour la commande du moteur.

Dans ce cas, la cassette comporte, avantageusement, un boîtier dans lequel la mémoire est fixée et, placés de manière amovible à l'intérieur de ce boîtier, un module médicament comprenant une poche destinée à contenir le liquide à injecter, un tuyau souple reliant la poche à l'extérieur et une pièce d'appui contre laquelle, lors de la rotation du rotor, les galets de celui-ci viennent tour à tour écraser le tuyau et pousser ainsi vers l'extérieur, par un mouvement péristaltique, le liquide contenu dans la poche.

Selon le mode de réalisation ci-dessus, les deux boîtiers comportent des organes d'accouplement de l'un à l'autre et sont dotés de pistes conductrices qui, lors de l'accouplement des boîtiers, se trouvent mises en communication de manière à interconnecter la mémoire et le microprocesseur.

De façon avantageuse, les données enregistrées dans la mémoire comportent, pour chaque étape du programme correspondant à un débit d'injection constant, les instants de début et de fin de l'étape ainsi que le nombre d'impulsions appliquées au moteur par unité de temps, déterminant la vitesse de rotation du rotor et donc le débit d'injection. Les données enregistrées dans la mémoire peuvent comporter, en plus, pour chaque bolus demandé par le patient, sa durée, le nombre d'impulsions motrices additionnelles par unité de temps, le nombre de bolus autorisés par jour et leur écart minimum.

La présente invention concerne également un équipement pour la programmation de la pompe définie ci-dessus, caractérisé en ce qu'il comporte:
- un ordinateur contenant, en mémoire, pour une pluralité de médicaments susceptibles d'être administrés par la pompe, le débit d'injection instantané maximum admissible, la quantité injectable maximum admissible par jour,
- fixée sur la poche, une étiquette d'identification du médicament qu'elle contient,
- des moyens de lecture de l'étiquette, reliés à l'ordinateur, et
- une interface d'échange de données, reliée à l'ordinateur et agencée pour le relier à la mémoire de la cassette.

Selon un mode de mise oeuvre préféré, l'ordinateur est programmé pour effectuer, à partir des informations livrées par l'étiquette et en réponse aux instructions fournies par un opérateur pour le programme d'injection désiré:
- la vérification que lesdites instructions respectent les valeurs admissibles contenues en mémoire,
- le calcul, pour chaque étape du programme correspondant à un débit d'injection constant, des instants de début et de fin de l'étape ainsi que du nombre d'impulsions appliquées au moteur par unité de temps, puis
- à la demande de l'opérateur, le transfert des valeurs calculées à la mémoire de la cassette.

Avantageusement l'ordinateur contient, en plus, pour certains, au moins, des médicaments susceptibles d'être administrés par la pompe, le nombre de bolus autorisés par jour, le volume maximum de chaque bolus et l'espace minimum entre deux bolus. Dans ce cas, l'ordinateur est programmé pour effectuer, en plus, le calcul, pour chaque bolus demandé par le patient, de sa durée et du nombre d'impulsions motrices additionnelles par unité de temps.

D'autres caractéristiques de l'invention ressortiront de la description qui va suivre, faite en regard du dessin annexé dans lequel:
- la figure 1 est une vue d'ensemble de la pompe selon invention, avant l'accouplement de la cassette au module pompe;
- la figure 2 représente la cassette sans son module médicament;
- la figure 3 représente le module médicament; et
- la figure 4 montre schématiquement l'équipement nécessaire pour la programmation de la pompe.

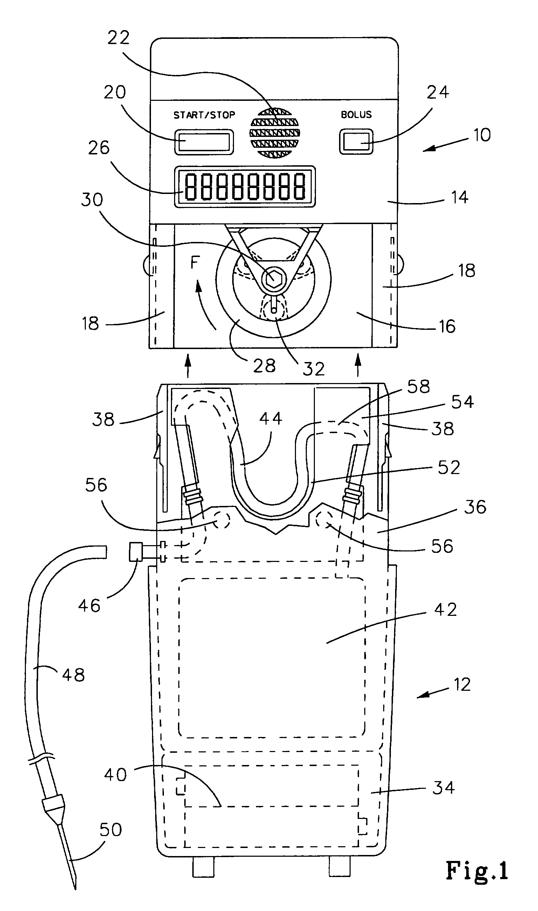

On se référera, tout d'abord, à la figure 1 qui montre une pompe à cassette programmable selon l'invention, formée d'un module pompe 10 et d'une cassette 12 s'accouplant de manière amovible.

La pompe proprement dite est décrite de manière très détaillée dans les deux documents précédemment cités. La présente description se limitera donc à ses éléments essentiels.

En bref, donc, le module pompe 10 comporte un boîtier en plastique rigide 14 dont la partie côté cassette possède seulement un fond 16 et deux coulisses latérales parallèles 18 servant à la mise en place de la cassette 12 à la manière d'un tiroir.

Sur sa face supérieure, le boîtier 14 comporte un bouton START/STOP 20 servant à commander le démarrage et l'arrêt de la pompe, un avertisseur d'alarme acoustique 22, un bouton BOLUS 24 servant à déclencher l'administration de doses additionnelles de médicament et un affichage LCD 26.

Le boîtier 14 laisse apparaître, entre ses deux coulisses 18, un rotor 28 monté en libre rotation autour d'un axe 30 fixé sur le fond 16 et entraîné en rotation au moyen d'un moteur pas à pas par l'intermédiaire d'un train d'engrenages (non visibles sur la figure). Le boîtier 14 abrite également un microprocesseur chargé de commander l'ensemble des éléments sus-mentionnés.

Le rotor 28 porte trois galets presseurs cylindriques 32 disposés à 120° l'un de l'autre et montés libres en rotation autour d'axes parallèles à l'axe 30.

La cassette 12, montrée séparée du module pompe 10, comporte un boîtier en plastique rigide 34 dont la partie côté module pompe possède seulement une face supérieure 36 et deux bras latéraux parallèles 38 destinés à s'insérer dans les coulisses 18 du module pompe 10. Le reste de la cassette 12 est occupé par deux piles 40 et une poche en plastique 42 remplie d'un médicament liquide, toutes disposées sous des couvercles.

La poche 42 est reliée à un tuyau souple en plastique 44 qui prend place entre les deux bras 38 et dont l'extrémité débouche à l'extérieur de la cassette où elle est obturée par un bouchon 46. C'est au moment de la mise en service de la pompe que ce bouchon sera enlevé pour permettre de brancher un tuyau flexible 48 se terminant par une aiguille d'injection 50.

Dans sa portion comprise entre les bras 38, le tuyau 44 est appliqué contre une pièce d'appui arrondie 52 en forme de U dont le rayon est légèrement supérieur à celui du cercle que parcourt la face externe des galets 32. La pièce 52 fait partie d'une plaque en plastique rigide 54 fixée sous la face supérieure 36 par enclipsage sur deux clavettes 56. C'est sur cette pièce en U 52 que, une fois les deux parties assemblées, lors de la rotation du rotor 28 dans le sens de la flèche F, ses trois galets presseurs 32 viennent tour à tour écraser le tuyau souple 44 et pousser ainsi vers l'extérieur, par un mouvement péristaltique, le liquide contenu dans la poche 42.

De chaque côté de la pièce d'appui 52, la plaque 54 est percée d'un canal 58 dans lequel prend place le tuyau 44.

La liaison électrique entre la cassette 12 et le module pompe 10 est assurée par des pistes conductrices (non représentées sur la figure 1) déposées sur leurs boîtiers respectifs.

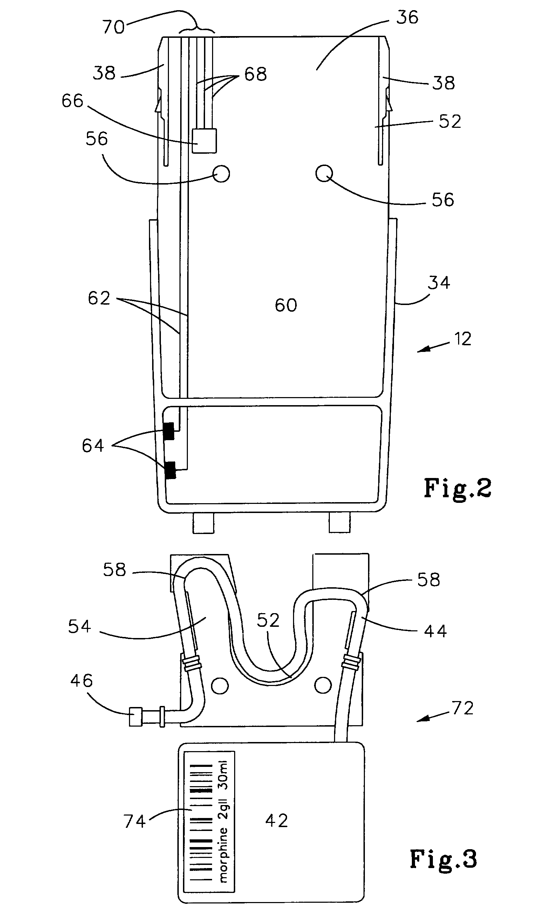

On se référera maintenant à la figure 2 qui montre la cassette 12, vue de dessous, avant la mise en place des piles 40, de la poche 42, de leur couvercle et de la plaque 54. On retrouve donc, sur cette figure, le boîtier 34, avec ses deux bras 38 et les deux clavettes 56 pour l'enclipsage de la plaque 54.

On voit alors que le fond 60 du boîtier comporte deux pistes conductrices 62 reliant les deux bornes 64 de connexion des piles à l'extrémité du boîtier côté module pompe. Le fond 60 comporte aussi un circuit intégré mémoire 66, de type connu sous la dénomination usuelle E2PROM, et un deuxième ensemble de pistes conductrices 68 reliant également la mémoire 66 à l'extrémité du boîtier, dotée de plages 70 qui, lors de l'assemblage de la cassette 12 au module pompe 10, viennent au contact de plages identiques ménagées sur son boîtier pour assurer l'interconnexion avec le microprocesseur.

Le rôle joué par la mémoire 66 va maintenant apparaître dans la description des opérations de programmation et de mise en fonction de la pompe selon l'invention.

Comme le montre la figure 3, on dispose initialement d'un module médicament 72, représenté vu de dessous, comprenant la poche 42 contenant le médicament à injecter, son tuyau flexible 44 obturé par le bouchon 46, la plaque rigide 54 avec la pièce d'appui 52 et les canaux 58 dans lesquels le tuyau prend place, tous ces éléments ayant déjà été montrés sur la figure 1. Cet ensemble, sorti du stock de pharmacie, est identifié par une étiquette de type code à barres 74, collée sur la poche 42 et comportant, en code et en clair, le type de médicament et son dosage (morphine 2g/l) ainsi que son volume (30 ml).

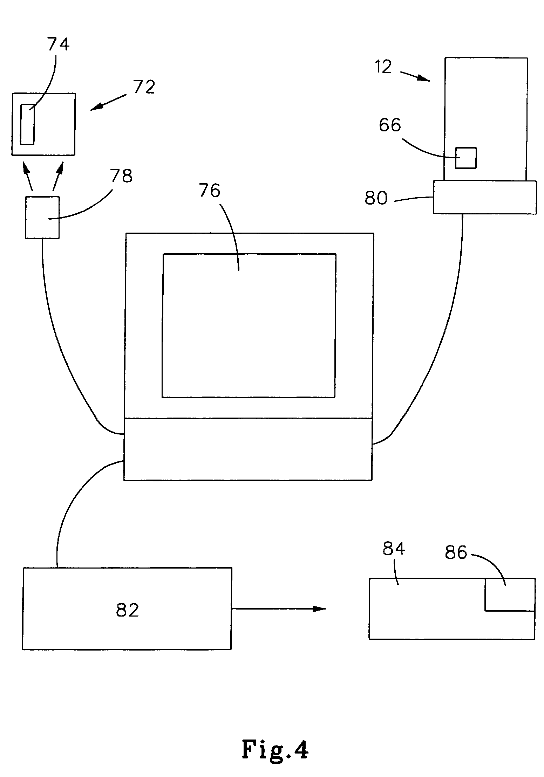

L'équipement nécessaire, schématisé sur la figure 4, se réduit à un ordinateur 76 et, reliés à lui, une tête de lecture de codes à barres 78, une interface d'échange de données 80 et une imprimante 82.

L'ordinateur 76 contient, en mémoire, pour les médicaments susceptibles d'être administrés par la pompe, les données suivantes:
- débit instantané maximum admissible,
- quantité injectable maximum admissible par jour, et
- nombre de bolus autorisés par jour (qui peut être 0), volume maximum autorisé de chaque bolus et espace minimum autorisé entre deux bolus.

L'interface 80, de type RS232, est dotée d'une fente conformée et dimensionnée pour recevoir l'extrémité de la cassette 12 dont les plages 70 viennent au contact de plages identiques assurant l'interconnexion.

La première opération est la lecture de l'étiquette 74 du module médicament 72 à l'aide de la tête 78. Les informations qu'elle contient (type de médicament et son dosage) déclenchent l'apparition d'un écran invitant à fournir à l'ordinateur 76 des indications relatives au patient (nom, adresse, ....) ainsi que les instructions relatives à l'administration du médicament concerné, dans les limites autorisées, soit:
- le programme d'injection: quels débits pendant quelles durées,
- le débit des bolus, leur nombre et espacement autorisés.

L'ordinateur 76 s'assure, à chaque introduction, que les valeurs fournies sont dans les limites admissibles contenues dans sa mémoire. Lorsqu'il est en possession de tous ces éléments validés, il calcule les paramètres qui doivent être utilisés par le microprocesseur du module pompe 10 pour que l'injection du médicament s'effectue conformément aux instructions données. Plus précisément, les paramètres calculés par l'ordinateur 76 sont:
- pour chaque étape du programme correspondant à un débit d'injection constant, les instants de début et de fin de l'étape ainsi que le nombre d'impulsions appliquées au moteur par unité de temps, ce nombre (qui peut être 0) déterminant la vitesse de rotation du rotor 30 et donc le débit d'injection;
- pour chaque bolus demandé par le patient, sa durée et le nombre d'impulsions motrices additionnelles par unité de temps.

Une fois ces paramètres calculés, la cassette vide 12, telle que représentée à la figure 2, est insérée dans l'interface 80. L'opérateur peut alors ordonner leur transfert à la mémoire E2PROM 66 en même temps qu'éventuellement, le nombre de bolus autorisés par jour et leur écart minimum.

Afin d'assurer la sécurité du système, la mémoire 66 retransmet les paramètres reçus à l'ordinateur 76 qui les compare aux paramètres envoyés, avant de produire, à travers l'imprimante 82, un document 84 contenant l'ensemble des informations introduites dans l'ordinateur et envoyées à la mémoire 66, en même temps qu'une étiquette 86 avec, par exemple, le nom du patient ainsi que la durée et la date du traitement. Cette étiquette peut être finalement collée sur la poche 42.

Il ne reste plus, alors, à l'opérateur qu'à placer l'ensemble 72, doté de son étiquette 86, dans la cassette 12 maintenant programmée. Si, comme cela est souhaitable, celle-ci comporte, à cet endroit, une portion transparente, l'étiquette restera visible afin de permettre, à tout moment, une vérification.

Le tout peut être ensuite envoyé ou remis au personnel soignant ou au patient qui, pour mettre la pompe en service, doit seulement glisser la cassette 12 dans le module pompe 10, enlever le bouchon 46, brancher à sa place le tuyau flexible 48 équipé de son aiguille d'injection 50 et enclencher la pompe en appuyant sur le bouton 20.

Ainsi, est proposée une pompe dont la mise en service se fait seulement par des opérations ultra simples ne nécessitant aucune formation spéciale puisque sa programmation est assurée automatiquement par la cassette lorsqu'elle est accouplée au module pompe. Il est évident, par ailleurs, que tout risque découlant d'une mauvaise programmation de la pompe au moment de sa mise en service est complètement exclu. Il faut remarquer, aussi, que la procédure de pré-progammation de la cassette par enregistrement d'instructions dans la mémoire E2PROM est particulièrement simple, conviviale et sûre. On notera, enfin, que cette pré-programmation, réalisée à l'aide d'un ordinateur et par un personnel qualifié, qui peut être le médecin cancérologue ou spécialiste de la douleur, offre de nombreuses possibilités de choix de programmes d'administration, ce qui est particulièrement important dans ces domaines.

## Revendications

1. Pompe programmable pour l'injection de médicaments, comportant deux parties accouplables de manière amovible, soit:
- une cassette (12) contenant un liquide à injecter, et
- un module pompe (10) doté de moyens d'actionnement pour faire passer le liquide de la cassette vers l'extérieur,
**caractérisée en ce que** la cassette comporte une mémoire électronique (66) dans laquelle ont été préalablement enregistrées des données relatives au programme d'injection prescrit et **en ce que**, lorsque les deux parties sont accouplées, lesdits moyens d'actionnement se trouvent électriquement connectés à la mémoire et répondent aux données qu'elle contient pour injecter le liquide selon le programme désiré.

2. Pompe selon la revendication 1, **caractérisée en ce que** le module pompe (10) est de type péristaltique et comporte un boîtier (14) et, à l'intérieur de celui-ci, un rotor (28) muni de galets presseurs (32), un moteur pas à pas pour l'entraînement dudit rotor et un microprocesseur pour la commande dudit moteur.

3. Pompe selon la revendication 2, **caractérisée en ce que** la cassette (12) comporte un boîtier (34) dans lequel est fixée ladite mémoire (66) et, placés de manière amovible à l'intérieur dudit boîtier, un module médicament (72) comprenant une poche (42) destinée à contenir le liquide à injecter, un tuyau souple (44) reliant ladite poche à l'extérieur et une pièce d'appui (52) contre laquelle, lors de la rotation dudit rotor, les galets de celui-ci viennent tour à tour écraser le tuyau et pousser ainsi vers l'extérieur, par un mouvement péristaltique, le liquide contenu dans la poche.

4. Pompe selon la revendication 3, **caractérisée en ce que** les deux boîtiers (14, 34) comportent des organes d'accouplement de l'un à l'autre et sont dotés de pistes conductrices (62, 68) qui, lors de l'accouplement des boîtiers, se trouvent mises en communication de manière à interconnecter ladite mémoire et ledit microprocesseur.

5. Pompe selon l'une des revendications 3 et 4, **caractérisée en ce que** les données enregistrées dans ladite mémoire (66) comportent, pour chaque étape du programme correspondant à un débit d'injection constant, les instants de début et de fin de l'étape ainsi que le nombre d'impulsions appliquées au moteur par unité de temps, déterminant la vitesse de rotation du rotor et donc le débit d'injection.

6. Pompe selon la revendication 5, **caractérisée en ce que** les données enregistrées dans ladite mémoire comportent, en plus, pour chaque bolus demandé par le patient, sa durée, le nombre d'impulsions motrices additionnelles par unité de temps, le nombre de bolus autorisés par jour et leur écart minimum.

7. Equipement pour la programmation de la pompe selon l'une des revendications 3 à 6, **caractérisé en ce qu'**il comporte:
- un ordinateur (76) contenant, en mémoire, pour une pluralité de médicaments susceptibles d'être administrés par la pompe, le débit d'injection instantané maximum admissible, la quantité injectable maximum admissible par jour,
- fixée sur la poche (42), une étiquette (74) d'identification du médicament qu'elle contient,
- des moyens de lecture (78) de ladite étiquette, reliés à l'ordinateur, et
- une interface d'échange de données (80), reliée à l'ordinateur et agencée pour le relier à la mémoire de la cassette.

8. Equipement selon la revendication 7, **caractérisé en ce que** ledit ordinateur est programmé pour effectuer, à partir des informations livrées par l'étiquette et en réponse aux instructions fournies par un opérateur pour le programme d'injection désiré:
- la vérification que lesdites instructions respectent les valeurs admissibles contenues en mémoire,
- le calcul, pour chaque étape du programme correspondant à un débit d'injection constant, des instants de début et de fin de l'étape ainsi que du nombre d'impulsions appliquées au moteur par unité de temps, puis
- à la demande de l'opérateur, le transfert des valeurs calculées à la mémoire de la cassette.

9. Equipement selon la revendication 7, **caractérisé en ce que** l'ordinateur contient, en plus, pour certains, au moins, desdits médicaments, le nombre de bolus autorisés par jour, le volume maximum de chaque bolus et l'espace minimum entre deux bolus.

10. Equipement selon les revendications 8 et 9, **caractérisé en ce que** l'ordinateur est programmé pour effectuer, en plus, le calcul, pour chaque bolus demandé par le patient, de sa durée et du nombre d'impulsions motrices additionnelles par unité de temps.
